# EUROPEAN PATENT APPLICATION

(11) **EP 1 344 536 A1**
(43) Date of publication of application: **17.09.2003**
(21) Application number: 01972606.6
(22) Date of filing: 28.09.2001
(51) Int. Cl.: A61K 48/00, A61K 38/17, A61K 39/395, A61K 31/711, C12N 15/85, A61P 43/00

(54) **METHOD OF REGULATING THE ACTIVITY OF EXPRESSION PRODUCT OF GENE TRANSFERRED INTO LIVING BODY**

(30) Priority: 24.11.2000 JP 2000358389
(71) Applicant: CHUGAI SEIYAKU KABUSHIKI KAISHA, Tokyo, 115-8543 (JP); Maruyama, Hiroki, Niigata-shi, Niigata 951-8124 (JP)
(72) Inventor: MARUYAMA, Hiroki, 706, Daiaparesu Igakucho, Niigata-shi, Niigata 951-8124 (JP); MIYAZAKI, Jun-ichi, Suita-shi, Osaka 565-0872 (JP); SUGAWA, Makoto, Gotenba-shi, Shizuoka 412-8513 (JP); HIGUCHI, Masato, Toshima-ku, Tokyo 171-8545 (JP)
(74) Representative: VOSSIUS & PARTNER
(86) International application number: JP0108575
(87) International publication number: WO02041922

(57) **Abstract**

The activity of an expression product of a gene introduced into a living body can be regulated by the coexistence of a protein (interfering substance) that interferes with the activity of the expression product. Receptors of the expression product and such may be used as the interfering substance. The activity of an overexpressed product of a gene introduced in gene therapy can be regulated. The administration of an interfering substance can be achieved by *in vivo* expression of the gene encoding the substance.

SEQUENCE LISTING

<110> MARUYAMA Hiroki
      CHUGAI SEIYAKU KABUSHIKI KAISHA

<120> METHOD FOR REGULATING THE ACTIVITY OF AN EXPRESSION PRODUCT
      OF A GENE INTRODUCED INTO A LIVING BODY

<130> C1-A0015P

<140>
<141>

<150> JP 2000-358389
<151> 2000-11-24

<160> 3

<170> PatentIn Ver. 2.1

<210> 1
<211> 5565
<212> DNA
<213> Homo sapiens

<400> 1

gtcgacattg attattgact agttattaat agtaatcaat tacggggtca ttagttcata 60
gcccatatat ggagttccgc gttacataac ttacggtaaa tggcccgcct ggctgaccgc 120
ccaacgaccc ccgcccattg acgtcaataa tgacgtatgt tcccatagta acgccaatag 180
ggactttcca ttgacgtcaa tgggtggact atttacggta aactgcccac ttggcagtac 240
atcaagtgta tcatatgcca agtacgcccc ctattgacgt caatgacggt aaatggcccg 300
cctggcatta tgcccagtac atgaccttat gggactttcc tacttggcag tacatctacg 360
tattagtcat cgctattacc atgggtcgag gtgagcccca cgttctgctt cactctcccc 420
atctcccccc cctccccacc cccaattttg tatttattta ttttttaatt attttgtgca 480
gcgatggggg cggggggggg gggggcgcgc gccaggcggg gcggggcggg gcgaggggcg 540
gggcggggcg aggcggagag gtgcggcggc agccaatcag agcggcgcgc tccgaaagtt 600
tccttttatg gcgaggcggc ggcggcggcg gccctataaa aagcgaagcg cgcggcgggc 660
gggagtcgct gcgttgcctt cgccccgtgc cccgctccgc gccgcctcgc gccgcccgcc 720
ccggctctga ctgaccgcgt tactcccaca ggtgagcggg cgggacggcc cttctcctcc 780
gggctgtaat tagcgcttgg tttaatgacg gctcgtttct tttctgtggc tgcgtgaaag 840
ccttaaaggg ctccgggagg gccctttgtg cggggggggag cggctcgggg ggtgcgtgcg 900
tgtgtgtgtg cgtggggagc gccgcgtgcg gcccgcgctg cccggcggct gtgagcgctg 960
cgggcgcggc gcggggcttt gtgcgctccg cgtgtgcgcg aggggagcgc ggccgggggc 1020
ggtgccccgc ggtgcggggg ggctgcgagg ggaacaaagg ctgcgtgcgg ggtgtgtgcg 1080
tgggggggtg agcagggggt gtgggcgcgg cggtcgggct gtaacccccc cctgcacccc 1140
cctccccgag ttgctgagca cggcccggct tcgggtgcgg ggctccgtgc ggggcgtggc 1200
gcggggctcg ccgtgccggg cggggggtgg cggcaggtgg gggtgccggg cggggcgggg 1260
ccgcctcggg ccggggaggg ctcgggggag gggcgcggcg gccccggagc gccggcggct 1320
gtcgaggcgc ggcgagccgc agccattgcc ttttatggta atcgtgcgag agggcgcagg 1380
gacttccttt gtcccaaatc tggcggagcc gaaatctggg aggcgccgcc gcaccccctc 1440
tagcgggcgc gggcgaagcg gtgcggcgcc ggcaggaagg aaatgggcgg ggagggcctt 1500
cgtgcgtcgc cgcgccgccg tccccttctc catctccagc ctcggggctg ccgcaggggg 1560

acggctgcct tcgggggga cggggcaggg cggggttcgg cttctggcgt gtgaccggcg 1620
gctctagagc ctctgctaac catgttcatg ccttcttctt tttcctacag ctcctgggca 1680
acgtgctggt tgttgtgctg tctcatcatt ttggcaaaga attccaattc caccatggac 1740
cacctcgggg cgtccctctg gccccaggtc ggctcccttt gtctcctgct cgctggggcc 1800
gcctgggcgc ccccgcctaa cctcccggac cccaagttcg agagcaaagc ggccttgctg 1860
gcggcccggg ggcccgaaga gcttctgtgc ttcaccgagc ggttggagga cttggtgtgt 1920
ttctgggagg aagcggcgag cgctggggtg ggcccgggca actacagctt ctcctaccag 1980
ctcgaggatg agccatggaa gctgtgtcgc ctgcaccagg ctcccacggc tcgtggtgcg 2040
gtgcgcttct ggtgttcgct gcctacagcc gacacgtcga gcttcgtgcc cctagagttg 2100
cgcgtcacag cagcctccgg cgctccgcga tatcaccgtg tcatccacat caatgaagta 2160
gtgctcctag acgcccccgt ggggctggtg gcgcggttgg ctgacgagag cggccacgta 2220
gtgttgcgct ggctcccgcc gcctgagaca cccatgacgt ctcacatccg ctacgaggtg 2280
gacgtctcgg ccggcaacgg cgcagggagc gtacagaggg tggagatcct ggagggccgc 2340
accgagtgtg tgctgagcaa cctgcggggc cggacgcgct acaccttcgc cgtccgcgcg 2400
cgtatggctg agccgagctt cggcggcttc tggagcgcct ggtcggagcc tgtgtcgctg 2460
ctgacgccta gcgactacaa agacgatgac gataaataag tcgaggaatt cactcctcag 2520
gtgcaggctg cctatcagaa ggtggtggct ggtgtggcca atgccctggc tcacaaatac 2580
cactgagatc tttttccctc tgccaaaaat tatggggaca tcatgaagcc ccttgagcat 2640
ctgacttctg gctaataaag gaaatttatt ttcattgcaa tagtgtgttg gaattttttg 2700
tgtctctcac tcggaaggac atatgggagg gcaaatcatt taaaacatca gaatgagtat 2760
ttggtttaga gtttggcaac atatgccata tgctggctgc catgaacaaa ggtggctata 2820
aagaggtcat cagtatatga aacagccccc tgctgtccat tccttattcc atagaaaagc 2880
cttgacttga ggttagattt tttttatatt ttgttttgtg ttattttttt ctttaacatc 2940
cctaaaattt tccttacatg ttttactagc cagatttttc ctcctctcct gactactccc 3000
agtcatagct gtccctcttc tcttatgaag atccctcgac ctgcagccca agcttggcgt 3060
aatcatggtc atagctgttt cctgtgtgaa attgttatcc gctcacaatt ccacacaaca 3120

tacgagccgg aagcataaag tgtaaagcct ggggtgccta atgagtgagc taactcacat 3180

taattgcgtt gcgctcactg cccgctttcc agtcgggaaa cctgtcgtgc cagcggatcc 3240

gcatctcaat tagtcagcaa ccatagtccc gcccctaact ccgcccatcc cgcccctaac 3300

tccgcccagt tccgcccatt ctccgcccca tggctgacta atttttttta tttatgcaga 3360

ggccgaggcc gcctcggcct ctgagctatt ccagaagtag tgaggaggct tttttggagg 3420

cctaggcttt tgcaaaaagc taacttgttt attgcagctt ataatggtta caaataaagc 3480

aatagcatca caaatttcac aaataaagca tttttttcac tgcattctag ttgtggtttg 3540

tccaaactca tcaatgtatc ttatcatgtc tggatccgct gcattaatga atcggccaac 3600

gcgcggggag aggcggtttg cgtattgggc gctcttccgc ttcctcgctc actgactcgc 3660

tgcgctcggt cgttcggctg cggcgagcgg tatcagctca ctcaaaggcg gtaatacggt 3720

tatccacaga atcaggggat aacgcaggaa agaacatgtg agcaaaaggc cagcaaaagg 3780

ccaggaaccg taaaaaggcc gcgttgctgg cgtttttcca taggctccgc ccccctgacg 3840

agcatcacaa aaatcgacgc tcaagtcaga ggtggcgaaa cccgacagga ctataaagat 3900

accaggcgtt tccccctgga agctccctcg tgcgctctcc tgttccgacc ctgccgctta 3960

ccggatacct gtccgccttt ctcccttcgg gaagcgtggc gctttctcaa tgctcacgct 4020

gtaggtatct cagttcggtg taggtcgttc gctccaagct gggctgtgtg cacgaacccc 4080

ccgttcagcc cgaccgctgc gccttatccg gtaactatcg tcttgagtcc aacccggtaa 4140

gacacgactt atcgccactg gcagcagcca ctggtaacag gattagcaga gcgaggtatg 4200

taggcggtgc tacagagttc ttgaagtggt ggcctaacta cggctacact agaaggacag 4260

tatttggtat ctgcgctctg ctgaagccag ttaccttcgg aaaaagagtt ggtagctctt 4320

gatccggcaa acaaaccacc gctggtagcg gtggtttttt tgtttgcaag cagcagatta 4380

cgcgcagaaa aaaaggatct caagaagatc ctttgatctt ttctacgggg tctgacgctc 4440

agtggaacga aaactcacgt taagggattt tggtcatgag attatcaaaa aggatcttca 4500

cctagatcct tttaaattaa aaatgaagtt ttaaatcaat ctaaagtata tatgagtaaa 4560

cttggtctga cagttaccaa tgcttaatca gtgaggcacc tatctcagcg atctgtctat 4620

ttcgttcatc catagttgcc tgactccccg tcgtgtagat aactacgata cgggagggct 4680

taccatctgg ccccagtgct gcaatgatac cgcgagaccc acgctcaccg gctccagatt 4740
tatcagcaat aaaccagcca gccggaaggg ccgagcgcag aagtggtcct gcaactttat 4800
ccgcctccat ccagtctatt aattgttgcc gggaagctag agtaagtagt tcgccagtta 4860
atagtttgcg caacgttgtt gccattgcta caggcatcgt ggtgtcacgc tcgtcgtttg 4920
gtatggcttc attcagctcc ggttcccaac gatcaaggcg agttacatga tcccccatgt 4980
tgtgcaaaaa agcggttagc tccttcggtc ctccgatcgt tgtcagaagt aagttggccg 5040
cagtgttatc actcatggtt atggcagcac tgcataattc tcttactgtc atgccatccg 5100
taagatgctt ttctgtgact ggtgagtact caaccaagtc attctgagaa tagtgtatgc 5160
ggcgaccgag ttgctcttgc ccggcgtcaa tacgggataa taccgcgcca catagcagaa 5220
ctttaaaagt gctcatcatt ggaaaacgtt cttcggggcg aaaactctca aggatcttac 5280
cgctgttgag atccagttcg atgtaaccca ctcgtgcacc caactgatct tcagcatctt 5340
ttactttcac cagcgtttct gggtgagcaa aaacaggaag gcaaaatgcc gcaaaaaagg 5400
gaataagggc gacacggaaa tgttgaatac tcatactctt cctttttcaa tattattgaa 5460
gcatttatca gggttattgt ctcatgagcg gatacatatt tgaatgtatt tagaaaaata 5520
aacaaatagg ggttccgcgc acatttcccc gaaaagtgcc acctg 5565

<210> 2
<211> 5504
<212> DNA
<213> Rattus norvegicus

<400> 2
gtcgacattg attattgact agttattaat agtaatcaat tacggggtca ttagttcata 60
gcccatatat ggagttccgc gttacataac ttacggtaaa tggcccgcct ggctgaccgc 120
ccaacgaccc ccgcccattg acgtcaataa tgacgtatgt tcccatagta acgccaatag 180

ggactttcca ttgacgtcaa tgggtggact atttacggta aactgcccac ttggcagtac 240
atcaagtgta tcatatgcca agtacgcccc ctattgacgt caatgacggt aaatggcccg 300
cctggcatta tgcccagtac atgaccttat gggactttcc tacttggcag tacatctacg 360
tattagtcat cgctattacc atgggtcgag gtgagcccca cgttctgctt cactctcccc 420
atctcccccc cctccccacc cccaattttg tatttattta ttttttaatt attttgtgca 480
gcgatggggg cggggggggg gggggcgcgc gccaggcggg gcggggcggg gcgaggggcg 540
gggcggggcg aggcggagag gtgcggcggc agccaatcag agcggcgcgc tccgaaagtt 600
tccttttatg gcgaggcggc ggcggcggcg gccctataaa aagcgaagcg cgcggcgggc 660
gggagtcgct gcgttgcctt cgccccgtgc cccgctccgc gccgcctcgc gccgcccgcc 720
ccggctctga ctgaccgcgt tactcccaca ggtgagcggg cgggacggcc cttctcctcc 780
gggctgtaat tagcgcttgg tttaatgacg gctcgtttct tttctgtggc tgcgtgaaag 840
ccttaaaggg ctccgggagg gccctttgtg cggggggggag cggctcgggg ggtgcgtgcg 900
tgtgtgtgtg cgtggggagc gccgcgtgcg gcccgcgctg cccggcggct gtgagcgctg 960
cgggcgcggc gcggggcttt gtgcgctccg cgtgtgcgcg aggggagcgc ggccgggggc 1020
ggtgccccgc ggtgcggggg ggctgcgagg ggaacaaagg ctgcgtgcgg ggtgtgtgcg 1080
tgggggggtg agcagggggt gtgggcgcgg cggtcgggct gtaacccccc cctgcacccc 1140
cctccccgag ttgctgagca cggcccggct tcgggtgcgg ggctccgtgc ggggcgtggc 1200
gcggggctcg ccgtgccggg cggggggtgg cggcaggtgg gggtgccggg cggggcgggg 1260
ccgcctcggg ccggggaggg ctcgggggag gggcgcggcg gccccggagc gccggcggct 1320
gtcgaggcgc ggcgagccgc agccattgcc ttttatggta atcgtgcgag agggcgcagg 1380
gacttccttt gtcccaaatc tggcggagcc gaaatctggg aggcgccgcc gcaccccctc 1440
tagcgggcgc gggcgaagcg gtgcggcgcc ggcaggaagg aaatgggcgg ggagggcctt 1500
cgtgcgtcgc cgcgccgccg tccccttctc catctccagc ctcggggctg ccgcaggggg 1560
acggctgcct tcggggggga cggggcaggg cggggttcgg cttctggcgt gtgaccggcg 1620
gctctagagc ctctgctaac catgttcatg ccttcttctt tttcctacag ctcctgggca 1680
acgtgctggt tgttgtgctg tctcatcatt ttggcaaaga attcctcgaa tcggagatgg 1740

gggtgcccga acgtcccacc ctgctgcttt tactatcctt gctactgatt cctctgggcc 1800
tcccagtcct ctgcgctccc ccacgcctca tttgcgacag tcgcgttctg gagaggtaca 1860
tcttggaggc caaggaggca gaaaatgtca caatgggctg tgcagaaggt cccagactga 1920
gtgagaatat taccgtccca gataccaaag tcaacttcta cgcttggaaa agaatgaagg 1980
tggaagaaca ggctgtagaa gtttggcaag gcctgtctct gctctcagaa gccatcctgc 2040
aggcccaggc tctgcaggcc aattcctccc agccaccaga gagtcttcag cttcatatag 2100
acaaagccat cagtgggcta cgtagcctca cttcactgct tcgggtgctg ggagctcaga 2160
aggaattgat gtcgcctcca gacgccaccc aagccgctcc actccgaaca ctcacagcgg 2220
atactttctg caagctcttc cgggtctact ccaacttcct ccgggggaaa ctgaagctgt 2280
acacggggga ggcctgcagg agaggggaca ggtgacctgc cactgccgtg tacccgccaa 2340
ctcgctcacc gtcactgtgt cacgccaacc ctccaccact cccaaccctc atcaaacggg 2400
gttgtttgtt accttcttac cggcctgtcc tacgcggcct cgaggaattc actcctcagg 2460
tgcaggctgc ctatcagaag gtggtggctg gtgtggccaa tgccctggct cacaaatacc 2520
actgagatct ttttccctct gccaaaaatt atggggacat catgaagccc cttgagcatc 2580
tgacttctgg ctaataaagg aaatttattt tcattgcaat agtgtgttgg aattttttgt 2640
gtctctcact cggaaggaca tatgggaggg caaatcattt aaaacatcag aatgagtatt 2700
tggtttagag tttggcaaca tatgccatat gctggctgcc atgaacaaag gtggctataa 2760
agaggtcatc agtatatgaa acagccccct gctgtccatt ccttattcca tagaaaagcc 2820
ttgacttgag gttagatttt ttttatattt tgttttgtgt tatttttttc tttaacatcc 2880
ctaaaatttt ccttacatgt tttactagcc agatttttcc tcctctcctg actactccca 2940
gtcatagctg tccctcttct cttatgaaga tccctcgacc tgcagcccaa gcttggcgta 3000
atcatggtca tagctgtttc ctgtgtgaaa ttgttatccg ctcacaattc cacacaacat 3060
acgagccgga agcataaagt gtaaagcctg gggtgcctaa tgagtgagct aactcacatt 3120
aattgcgttg cgctcactgc ccgctttcca gtcgggaaac ctgtcgtgcc agcggatccg 3180
catctcaatt agtcagcaac catagtcccg cccctaactc cgcccatccc gcccctaact 3240
ccgcccagtt ccgcccattc tccgccccat ggctgactaa ttttttttat ttatgcagag 3300

gccgaggccg cctcggcctc tgagctattc cagaagtagt gaggaggctt ttttggaggc 3360

ctaggctttt gcaaaaagct aacttgttta ttgcagctta taatggttac aaataaagca 3420

atagcatcac aaatttcaca aataaagcat ttttttcact gcattctagt tgtggtttgt 3480

ccaaactcat caatgtatct tatcatgtct ggatccgctg cattaatgaa tcggccaacg 3540

cgcggggaga ggcggtttgc gtattgggcg ctcttccgct tcctcgctca ctgactcgct 3600

gcgctcggtc gttcggctgc ggcgagcggt atcagctcac tcaaaggcgg taatacggtt 3660

atccacagaa tcaggggata acgcaggaaa gaacatgtga gcaaaaggcc agcaaaaggc 3720

caggaaccgt aaaaaggccg cgttgctggc gtttttccat aggctccgcc cccctgacga 3780

gcatcacaaa aatcgacgct caagtcagag gtggcgaaac ccgacaggac tataaagata 3840

ccaggcgttt ccccctggaa gctccctcgt gcgctctcct gttccgaccc tgccgcttac 3900

cggatacctg tccgcctttc tcccttcggg aagcgtggcg ctttctcaat gctcacgctg 3960

taggtatctc agttcggtgt aggtcgttcg ctccaagctg ggctgtgtgc acgaaccccc 4020

cgttcagccc gaccgctgcg ccttatccgg taactatcgt cttgagtcca acccggtaag 4080

acacgactta tcgccactgg cagcagccac tggtaacagg attagcagag cgaggtatgt 4140

aggcggtgct acagagttct tgaagtggtg gcctaactac ggctacacta gaaggacagt 4200

atttggtatc tgcgctctgc tgaagccagt taccttcgga aaaagagttg gtagctcttg 4260

atccggcaaa caaaccaccg ctggtagcgg tggttttttt gtttgcaagc agcagattac 4320

gcgcagaaaa aaaggatctc aagaagatcc tttgatcttt tctacggggt ctgacgctca 4380

gtggaacgaa aactcacgtt aagggatttt ggtcatgaga ttatcaaaaa ggatcttcac 4440

ctagatcctt ttaaattaaa aatgaagttt taaatcaatc taaagtatat atgagtaaac 4500

ttggtctgac agttaccaat gcttaatcag tgaggcacct atctcagcga tctgtctatt 4560

tcgttcatcc atagttgcct gactccccgt cgtgtagata actacgatac gggagggctt 4620

accatctggc cccagtgctg caatgatacc gcgagaccca cgctcaccgg ctccagattt 4680

atcagcaata aaccagccag ccggaagggc cgagcgcaga agtggtcctg caactttatc 4740

cgcctccatc cagtctatta attgttgccg ggaagctaga gtaagtagtt cgccagttaa 4800

tagtttgcgc aacgttgttg ccattgctac aggcatcgtg gtgtcacgct cgtcgtttgg 4860

tatggcttca ttcagctccg gttcccaacg atcaaggcga gttacatgat cccccatgtt 4920

gtgcaaaaaa gcggttagct ccttcggtcc tccgatcgtt gtcagaagta agttggccgc 4980

agtgttatca ctcatggtta tggcagcact gcataattct cttactgtca tgccatccgt 5040

aagatgcttt tctgtgactg gtgagtactc aaccaagtca ttctgagaat agtgtatgcg 5100

gcgaccgagt tgctcttgcc cggcgtcaat acgggataat accgcgccac atagcagaac 5160

tttaaaagtg ctcatcattg gaaaacgttc ttcggggcga aaactctcaa ggatcttacc 5220

gctgttgaga tccagttcga tgtaacccac tcgtgcaccc aactgatctt cagcatcttt 5280

tactttcacc agcgtttctg ggtgagcaaa aacaggaagg caaaatgccg caaaaaaggg 5340

aataagggcg acacggaaat gttgaatact catactcttc ctttttcaat attattgaag 5400

catttatcag ggttattgtc tcatgagcgg atacatattt gaatgtattt agaaaaataa 5460

acaaataggg gttccgcgca catttccccg aaaagtgcca cctg 5504

<210> 3

<211> 6256

<212> DNA

<213> Unknown Organism

<220>

<223> Description of Unknown Organism:Chimeric gene

<220>

<223> pCAGGS-hSEPOR2 rat IgG1 Fc cDNA

<400> 3
   gtcgacattg attattgact agttattaat agtaatcaat tacggggtca ttagttcata 60
   gcccatatat ggagttccgc gttacataac ttacggtaaa tggcccgcct ggctgaccgc 120
   ccaacgaccc ccgcccattg acgtcaataa tgacgtatgt tcccatagta acgccaatag 180
   ggactttcca ttgacgtcaa tgggtggact atttacggta aactgcccac ttggcagtac 240
   atcaagtgta tcatatgcca agtacgcccc ctattgacgt caatgacggt aaatggcccg 300
   cctggcatta tgcccagtac atgaccttat gggactttcc tacttggcag tacatctacg 360
   tattagtcat cgctattacc atgggtcgag gtgagcccca cgttctgctt cactctcccc 420
   atctcccccc cctccccacc cccaattttg tatttattta ttttttaatt attttgtgca 480
   gcgatggggg cggggggggg gggggcgcgc gccaggcggg gcggggcggg gcgaggggcg 540
   gggcggggcg aggcggagag gtgcggcggc agccaatcag agcggcgcgc tccgaaagtt 600
   tccttttatg gcgaggcggc ggcggcggcg gccctataaa aagcgaagcg cgcggcgggc 660
   gggagtcgct gcgttgcctt cgccccgtgc cccgctccgc gccgcctcgc gccgcccgcc 720
   ccggctctga ctgaccgcgt tactcccaca ggtgagcggg cgggacggcc cttctcctcc 780
   gggctgtaat tagcgcttgg tttaatgacg gctcgtttct tttctgtggc tgcgtgaaag 840
   ccttaaaggg ctccgggagg gccctttgtg cgggggggag cggctcgggg ggtgcgtgcg 900
   tgtgtgtgtg cgtggggagc gccgcgtgcg gcccgcgctg cccggcggct gtgagcgctg 960
   cgggcgcggc gcggggcttt gtgcgctccg cgtgtgcgcg aggggagcgc ggccgggggc 1020
   ggtgccccgc ggtgcggggg ggctgcgagg ggaacaaagg ctgcgtgcgg ggtgtgtgcg 1080
   tgggggggtg agcagggggt gtgggcgcgg cggtcgggct gtaacccccc cctgcacccc 1140
   cctccccgag ttgctgagca cggcccggct tcgggtgcgg ggctccgtgc ggggcgtggc 1200
   gcggggctcg ccgtgccggg cggggggtgg cggcaggtgg gggtgccggg cggggcgggg 1260
   ccgcctcggg ccggggaggg ctcgggggag gggcgcggcg gccccggagc gccggcggct 1320
   gtcgaggcgc ggcgagccgc agccattgcc ttttatggta atcgtgcgag agggcgcagg 1380
   gacttccttt gtcccaaatc tggcggagcc gaaatctggg aggcgccgcc gcaccccctc 1440
   tagcgggcgc gggcgaagcg gtgcggcgcc ggcaggaagg aaatgggcgg ggagggcctt 1500
   cgtgcgtcgc cgcgccgccg tccccttctc catctccagc ctcggggctg ccgcaggggg 1560
   acggctgcct tcggggggga cggggcaggg cggggttcgg cttctggcgt gtgaccggcg 1620
   gctctagagc ctctgctaac catgttcatg ccttcttctt tttcctacag ctcctgggca 1680
   acgtgctggt tgttgtgctg tctcatcatt ttggcaaaga attcatttaa ataattccac 1740
   catggaccac ctcggggcgt ccctctggcc ccaggtcggc tccctttgtc tcctgctcgc 1800
   tggggccgcc tgggcgcccc cgcctaacct cccggacccc aagttcgaga gcaaagcggc 1860
   cttgctggcg gcccgggggc ccgaagagct tctgtgcttc accgagcggt tggaggactt 1920
   ggtgtgtttc tgggaggaag cggcgagcgc tggggtgggc ccgggcaact acagcttctc 1980
   ctaccagctc gaggatgagc catggaagct gtgtcgcctg caccaggctc ccacggctcg 2040
   tggtgcggtg cgcttctggt gttcgctgcc tacagccgac acgtcgagct tcgtgcccct 2100
   agagttgcgc gtcacagcag cctccggcgc tccgcgatat caccgtgtca tccacatcaa 2160
   tgaagtagtg ctcctagacg cccccgtggg gctggtggcg cggttggctg acgagagcgg 2220
   ccacgtagtg ttgcgctggc tcccgccgcc tgagacaccc atgacgtctc acatccgcta 2280
   cgaggtggac gtctcggccg gcaacggcgc agggagcgta cagagggtgg agatcctgga 2340
   gggccgcacc gagtgtgtgc tgagcaacct gcggggccgg acgcgctaca ccttcgccgt 2400
   ccgcgcgcgt atggctgagc cgagcttcgg cggcttctgg agcgcctggt cggagcctgt 2460
   gtcgctgctg acgcctagcg acctggacgc ggccgccgtg cccagaaact gtggaggtga 2520
   ttgcaagcct tgtatatgta caggctcaga agtatcatct gtcttcatct tccccccaaa 2580
   gcccaaagat gtgctcacca tcactctgac tcctaaggtc acgtgtgttg tggtagacat 2640
   tagccaggac gatcccgagg tccatttcag ctggtttgta gatgacgtgg aagtccacac 2700
   agctcagact cgaccaccag aggagcagtt caacagcact ttccgctcag tcagtgaact 2760
   ccccatcctg caccaggact ggctcaatgg caggacgttc agatgcaagg tcaccagtgc 2820
   agctttccca tcccccatcg agaaaaccat ctccaaaccc gaaggcagaa cacaagttcc 2880
   gcatgtatac accatgtcac ctaccaagga agagatgatc cagaatgaag tcagtatcac 2940
   ctgcatggta aaaggcttct atcccccaga catttatgtg gagtggcaga tgaacggcca 3000
   gccacaggaa aactacaaga acactccacc tacgatggac acagatggga gttacttcct 3060
   ctacagcaag ctcaatgtga agaaggaaaa atggcagcag ggaaacacgt tcacgtgttc 3120
   tgtgctgcat gaaggcctgc acaaccacca tactgagaag agtctctccc actctccggg 3180
   taaatgaccc cagagtgaat tcactcctca ggtgcaggct gcctatcaga aggtggtggc 3240
   tggtgtggcc aatgccctgg ctcacaaata ccactgagat ctttttccct ctgccaaaaa 3300
   ttatggggac atcatgaagc cccttgagca tctgacttct ggctaataaa ggaaatttat 3360
   tttcattgca atagtgtgtt ggaatttttt gtgtctctca ctcggaagga catatgggag 3420
   ggcaaatcat ttaaaacatc agaatgagta tttggtttag agtttggcaa catatgccat 3480
   atgctggctg ccatgaacaa aggtggctat aaagaggtca tcagtatatg aaacagcccc 3540
   ctgctgtcca ttccttattc catagaaaag ccttgacttg aggttagatt ttttttatat 3600
   tttgttttgt gttatttttt tctttaacat ccctaaaatt ttccttacat gttttactag 3660
   ccagattttt cctcctctcc tgactactcc cagtcatagc tgtccctctt ctcttatgaa 3720
   gatccctcga cctgcagccc aagcttggcg taatcatggt catagctgtt tcctgtgtga 3780
   aattgttatc cgctcacaat tccacacaac atacgagccg gaagcataaa gtgtaaagcc 3840
   tggggtgcct aatgagtgag ctaactcaca ttaattgcgt tgcgctcact gcccgctttc 3900
   cagtcgggaa acctgtcgtg ccagcggatc cgcatctcaa ttagtcagca accatagtcc 3960
   cgcccctaac tccgcccatc ccgcccctaa ctccgcccag ttccgcccat tctccgcccc 4020
   atggctgact aatttttttt atttatgcag aggccgaggc cgcctcggcc tctgagctat 4080
   tccagaagta gtgaggaggc ttttttggag gcctaggctt ttgcaaaaag ctaacttgtt 4140
   tattgcagct tataatggtt acaaataaag caatagcatc acaaatttca caaataaagc 4200
   atttttttca ctgcattcta gttgtggttt gtccaaactc atcaatgtat cttatcatgt 4260
   ctggatccgc tgcattaatg aatcggccaa cgcgcgggga gaggcggttt gcgtattggg 4320
   cgctcttccg cttcctcgct cactgactcg ctgcgctcgg tcgttcggct gcggcgagcg 4380
   gtatcagctc actcaaaggc ggtaatacgg ttatccacag aatcagggga taacgcagga 4440
   aagaacatgt gagcaaaagg ccagcaaaag gccaggaacc gtaaaaaggc cgcgttgctg 4500
   gcgtttttcc ataggctccg cccccctgac gagcatcaca aaaatcgacg ctcaagtcag 4560
   aggtggcgaa acccgacagg actataaaga taccaggcgt ttccccctgg aagctccctc 4620
   gtgcgctctc ctgttccgac cctgccgctt accggatacc tgtccgcctt tctcccttcg 4680
   ggaagcgtgg cgctttctca atgctcacgc tgtaggtatc tcagttcggt gtaggtcgtt 4740
   cgctccaagc tgggctgtgt gcacgaaccc cccgttcagc ccgaccgctg cgccttatcc 4800
   ggtaactatc gtcttgagtc caacccggta agacacgact tatcgccact ggcagcagcc 4860
   actggtaaca ggattagcag agcgaggtat gtaggcggtg ctacagagtt cttgaagtgg 4920
   tggcctaact acggctacac tagaaggaca gtatttggta tctgcgctct gctgaagcca 4980
   gttaccttcg gaaaaagagt tggtagctct tgatccggca aacaaaccac cgctggtagc 5040
   ggtggttttt ttgtttgcaa gcagcagatt acgcgcagaa aaaaaggatc tcaagaagat 5100
   cctttgatct tttctacggg gtctgacgct cagtggaacg aaaactcacg ttaagggatt 5160
   ttggtcatga gattatcaaa aaggatcttc acctagatcc ttttaaatta aaaatgaagt 5220
   tttaaatcaa tctaaagtat atatgagtaa acttggtctg acagttacca atgcttaatc 5280
   agtgaggcac ctatctcagc gatctgtcta tttcgttcat ccatagttgc ctgactcccc 5340
   gtcgtgtaga taactacgat acgggagggc ttaccatctg gccccagtgc tgcaatgata 5400
   ccgcgagacc cacgctcacc ggctccagat ttatcagcaa taaaccagcc agccggaagg 5460
   gccgagcgca gaagtggtcc tgcaacttta tccgcctcca tccagtctat taattgttgc 5520
   cgggaagcta gagtaagtag ttcgccagtt aatagtttgc gcaacgttgt tgccattgct 5580
   acaggcatcg tggtgtcacg ctcgtcgttt ggtatggctt cattcagctc cggttcccaa 5640
   cgatcaaggc gagttacatg atcccccatg ttgtgcaaaa aagcggttag ctccttcggt 5700
   cctccgatcg ttgtcagaag taagttggcc gcagtgttat cactcatggt tatggcagca 5760
   ctgcataatt ctcttactgt catgccatcc gtaagatgct tttctgtgac tggtgagtac 5820
   tcaaccaagt cattctgaga atagtgtatg cggcgaccga gttgctcttg cccggcgtca 5880
   atacgggata ataccgcgcc acatagcaga actttaaaag tgctcatcat tggaaaacgt 5940
   tcttcggggc gaaaactctc aaggatctta ccgctgttga gatccagttc gatgtaaccc 6000
   actcgtgcac ccaactgatc ttcagcatct tttactttca ccagcgtttc tgggtgagca 6060
   aaaacaggaa ggcaaaatgc cgcaaaaaag ggaataaggg cgacacggaa atgttgaata 6120
   ctcatactct tcctttttca atattattga agcatttatc agggttattg tctcatgagc 6180
   ggatacatat ttgaatgtat ttagaaaaat aaacaaatag gggttccgcg cacatttccc 6240
   cgaaaagtgc cacctg 6256

## Description

### Technical Field

The present invention relates to a method for regulating the activity of an expression product of a gene artificially introduced into a living body.

### Background Art

Human gene therapy was tried for the first time in 1990 in the US, and thereafter has been widely conducted in many countries. It is expected that, once the background of diseases are understood at the gene level, gene therapy can be used for treating many diseases by regulating the expression of the genes causing the diseases. The most-widely conducted gene therapy at present is a type of therapy wherein the activity of a protein, which is deficient due to abnormalities in a gene, is compensated by introducing a normal gene. An example of this type of gene therapy is a therapeutic method for adenosine deaminase (ADA) deficiency, in which an ADA expression vector is introduced. Similar gene therapies include the introduction of normal p53 into cancer cells. This gene therapy attempts to treat cells in which cancerization cannot be suppressed due to mutations in the p53 gene by the action of normal p53.

In early gene therapies, the expression level of the artificially introduced gene was often lower than expected. Therefore, investigations into means to accomplish stronger expression of the introduced gene by improving vector and methods for introducing the gene have attracted attention as a new subject. However, with the progress of these studies, some are worried about a long-term increase in the expression of the introduced gene at significantly higher levels than the physiological level. The expression mechanism of an artificially introduced gene is not accompanied by a feedback mechanism that suppresses overexpression. Thus, a strong expression system always bears the problem of overexpression. Maintained expression of an artificially introduced gene may cause side effects during therapy.

For example, a type of hematopoietic hormone, erythropoietin (abbreviated hereinafter as EPO), is effective for the treatment of anemia. However, excessive EPO may cause plethora. Gene therapy using vascular endothelium growth factor (VEGF) is carried out to treat pain during walking (intermittent claudication), arteriosclerosis obliterans showing histological damage in the lower limb, ischemic cardiac diseases including chronic cardiac failure, restenosis, and such. In gene therapy of angiogenesis by VEGF, excessive VEGF may cause angiogenesis in many other sites in addition to the target site. Upon acceleration of angiogenesis, the possibility of abnormal development of tissues, risks of increase in malignant tumors in tumor bearing patients, and such may arise. Therefore, the expression level of an introduced gene and the activity of an expression product need to be controllable for future gene therapy.

An example of a method for regulating the expression level of an introduced gene includes the method wherein a regulatory region reacting with a drug is integrated into a vector to be introduced and then administering the drug (Ye et al., Science, 1999, 283, 88-91) . According to this method, however, problems arise, such as side effects caused by the administered drug. Therefore, there is a need for safer and more reliable methods for regulating the expressed gene.

Meanwhile, various cytokines have recently been revealed to be involved in diseases, and clinical application of anticytokine therapy, such as administration of soluble chimeric receptors or antibodies against these cytokines, is in progress and already has accomplished results (Feldman AM et al., The role of tumor necrosis factor in the pathophysiology of heart failure, J. American College of Cardiology, 2000, 35, 537-544; Boehme MW & Gao IK, Present importance of direct immunologically based intervention strategies using anticytokines in rheumatoid arthritis, Zeitschrift fur Rheumatologie, 1999, 58, 251-166; Choy EH et al., Monoclonal antibody therapy in rheumatoid arthritis, British J. Rheumatology, 1998, 37, 484-490).

For example, chimeric receptors of tumor necrosis factor-a (TNF-α) or antibodies against TNF-α have been administered against TNF which is indicated to be involved in Crohn's disease, heart diseases (Feldman AM et al., The role of tumor necrosis factor in the pathophysiology of heart failure, J. American College of Cardiology, 2000, 35, 537-544), and rheumatism (Ohshima S et al., Long-term follow-up of the changes in circulating cytokines, soluble cytokine receptors, and white blood cell subset counts in patients with rheumatoid arthritis (RA) after monoclonal anti-TNF α antibody therapy, J. Clin. Immunology, 1999, 19, 305-313; Choy EH et al., Monoclonal antibody therapy in rheumatoid arthritis, British J. Rheumatology, 1998, 37, 484-490) , and have been achieving clinically effective results. Furthermore, an inhibitory effect on hepatic fibrosis in an animal model was obtained by the administration of chimeric IgG-soluble receptor comprising an extracellular portion of TGF-β type II receptor indicated to be involved in fibrosis (George J et al., *In vivo* inhibition of rat stellate cell activation by soluble transforming growth factor β type II receptor: a potential new therapy for hepatic fibrosis, Proc. Natl. Acad. Sci. USA, 1999, 96, 12719-12724). In these reports, components which interfere with the activity of cytokines are administered in order to regulate the activity of the cytokines that are the cause of diseases.

In gene therapy wherein an adenovirus vector is administered, anti-CD4 monoclonal antibodies have also been administered to animals to suppress the immune response against the virus. As a result, effects such as sustained reporter expression of the introduced gene, decrease in the antibody titer against the virus, and suppression of IFN-γ increase have been achieved (Schroeder G et al., Immune response after adenoviral gene transfer in syngeneic heart transplants: effects of anti-CD-4 monoclonal antibody therapy, Transplantation, 2000, 70, 191-198). These results suggest the effectiveness of the anti-CD4 antibody therapy in organ transplantation, etc.

Furthermore, the administration of an antibody against CD45, involved in the activation of lymphocytes, has been shown to inhibit the onset of experimental allergic encephalitis (EAE) , suppress the proliferation of T cells, and significantly suppress the production of not only TNF-α but also Th-1 cytokines, such as IFN-γ and IL-2 (Schiffenbauer J et, al., Prevention of experimental allergic encephalomyelitis by an antibody to CD45RB, Cellular Immunology, 1998, 190, 173-182). However, these methods, wherein an antibody or a recombinant type protein of a soluble receptor against a target cytokine is administered, are considered to have problems, such as reduction in the effect of repeated administration due to antibody production against administered antibody or recombinant protein and costs of the recombinant protein.

### Disclosure of the Invention

The objective of the present invention is to provide a method for regulating the activity of an expression product of a gene artificially introduced into a living body.

The feedback mechanism inherent in a living body does not function on a gene artificially introduced into the living body. Accordingly, the present inventors conceived that a certain kind of feedback mechanism could be realized by administering a compound capable of regulating the activity of an artificially introduced protein *in vivo*.

Finally, the present inventors revealed that the activity of an expression product of a gene artificially introduced into a living body can be regulated by allowing a substance that interferes with the activity of the expression product to coexist with the gene *in vivo*, and completed the present invention. Specifically, the present invention relates to a method for regulating the activity of an expression product of a gene introduced into a living body, as well as a vector used therefor, as follows:
[1] a method for regulating the activity of an expression product of a gene introduced into a living body involving the coexistence of a protein that interferes with the activity of the expression product;
[2] the method according to [1], wherein the coexistence of the protein that interferes with the activity of the expression product is achieved by expressing a gene encoding the protein in the same living body;
[3] the method according to [2], wherein the gene encoding the protein that interferes with the activity of the expression product is introduced into the living body via an expression vector consisting of a naked DNA;
[4] the method according to [3], wherein the expression vector is introduced into the living body by electroporation;
[5] the method according to [1], wherein the protein that interferes with the activity of the expression product is a protein selected from the group consisting of: a receptor of the expression product, an activity-neutralizing antibody of the expression product, and a protein containing an active site of the receptor or the antibody;
[6] the method according to [2], wherein the gene encoding the protein that interferes with the activity of the expression product is expressed under the control of an inducible promoter;
[7] a method for regulating the activity of an expression product of a gene introduced in gene therapy by the method according to [1];
[8] the method according to [7] , wherein the introduced gene encodes a protein which belongs to a group selected from blood proteins , cytokines, and hormones;
[9] the method according to [8] , wherein the protein belonging to a group selected from blood proteins, cytokines, and hormones is selected from the group consisting of:
   erythropoietin, granulocyte-colony stimulating factor, granulocyte-macrophage colony stimulating factor, stem cell factor, thrombopoietin, tumor necrosis factor-α, transforming growth factor-β, CD4, CD45, bone morphogenetic protein, interferon-α, interferon-γ, interleukin-1, interleukin-2, interleukin-6, interleukin-11, interleukin-12, epidermal growth factor, acidic fibroblast growth factor, basic fibroblast growth factor, fibroblast growth factor-I, platelet derived growth factor, vascular endothelial growth factor, nerve growth factor, brain derived neurotrophic factor, insulin-like growth factor-I, insulin-like growth factor-II, human growth hormone, parathyroid hormone, angiostatin, pigment epithelial cell derived factor, and hepatocyte growth factor;
[10] the method according to [9], wherein the protein is erythropoietin;
[11] the method according to [10], wherein the protein that interferes with the activity of the expression product is a solubilized erythropoietin receptor;
[12] the method according to [1], wherein the protein that interferes with the activity of the expression product is a fusion protein with anotherprotein;
[13] an expression vector for regulating the activity of an expression product of a gene introduced into a living body, which can express a protein that interferes with the activity of the expression product in the same living body;
[14] the expression vector according to [13], wherein the protein that interferes with the activity of the expression product is a fusion protein with another protein;
[15] a composition for regulating the activity of an expression product of a gene introduced into a living body, which comprises the expression vector of [13] as an active ingredient;
[16] use of an expression vector that can express a protein which interferes with the activity of an expression product of a gene introduced into a living body in the same living body, in the production of a composition for. regulating the activity of the expression product of a gene introduced into a living body; and
[17] the use of the expression vector according to [16] , wherein the protein that interferes with the activity of the expression product is a fusion protein with another protein.

Herein, the phrase "gene introduced into a living body" refers to any gene which is artificially introduced into a living body for any purpose. For example, a representative gene includes those that are artificially introduced aiming gene therapy. In addition to therapeutic purposes, the introduction of a foreign gene is often attempted to introduce traits not inherent in a subject organism. For example, a gene introduced for the purpose of producing a useful protein in an animal is also included as the gene artificially introduced into a living body in the present invention.

This invention is particularly useful for genes whose purpose of introduction is achieved by expressing the gene product thereof in the blood of animal. As compared with locally limited overexpression of a product, an expression product existing in blood exerts systemic influence. Therefore, regulation of the activity of a protein encoded by a gene that is artificially introduced into a living body becomes particularly important when the gene expression product is produced into the blood of an animal. In the present invention, genes that are introduced to supply their expression products into blood include genes encoding proteins belonging to blood proteins, cytokines, or hormones. The term "blood protein" refers to various protein contained in blood, such as albumin, globulin, ferritin, and various enzymes. Examples of cytokines and hormones specifically include the following proteins:
erythropoietin (EPO);
granulocyte-colony stimulating factor (G-CSF);
granulocyte-macrophage colony stimulating factor (GM-CSF);
stem cell factor (SCF);
thrombopoietin (TPO);
tumor necrosis factor-α (TNF-α);
transforming growth factor-β (TGF-β);
CD4;
CD45;
bone morphogenetic protein (BMP);
interferon-α (IFN-α) ;
interferon-γ (IFN-γ);
interleukin-1 (IL-1) ;
interleukin-2 (IL-2);
interleukin-6 (IL-6);
interleukin-11 (IL-11);
interleukin-12 (IL-12);
epidermal growth factor (EGF);
acidic fibroblast growth factor (aFGF) ;
basic fibroblast growth factor (bFGF);
fibroblast-growth factor-I (FGF-I);
platelet derived growth factor (PDGF);
vascular endothelial growth factor (VEGF);
nerve growth factor (NGF) ;
brain derived neurotrophic factor (BDNF);
insulin-like growth factor-I (IGF-I);
insulin-like growth factor-II (IGF-II);
human growth hormone (hGH);
parathyroid hormone (PTH);
angiostatin;
pigment epithelial cell derived factor (PEDF); and hepatocyte growth factor (HGF).

Herein, the phrase "protein that interferes with the activity of a gene expression product" refers to proteins that can modify the activity of the gene expression product through a certain mechanism. The protein that interferes with the activity, herein, is referred to as the "interfering substance". Furthermore, "regulation" as used in the instant specification means to modify the activity of the gene expression product to a desired level by altering the amount of the interfering substance. The term "regulation" herein includes'not only down-regulation but also up-regulation of the activity. Specifically, interfering substances include proteins that interfere with the activity through binding, decomposition, competition, and such. More specifically, the interfering substances include receptors of the gene expression product, antibodies capable of inhibiting the binding between the gene expression product and its receptor by blocking a receptor-binding moiety of the gene expression product, and proteins acting as an antagonist of the gene expression product.

For example, for EPO, inferring substances of the present invention include EPO receptor, antibodies capable of inhibiting the binding between EPO and its receptor by blocking a receptor-binding moiety of EPO, and proteins acting as an antagonist of EPO. Similarly for other cytokines, such as VEGF, receptors whose ligand is the cytokine, or antibodies thereto can be used as the interfering substance.

These proteins can be active fragments thereof or fusion substances of an active fragment with other molecules, so long as they can interfere with the activity of the gene expression product. For example, a ligand-binding region of a receptor or a fusion protein containing the binding region can be used as an interfering substance. Furthermore, a fragment of an antibody containing its antigen-binding region, or a single-strand antibody can be also used. A fusion substance includes a fusion of the receptor and an antibody Fc region. By fusing the protein with the antibody Fc region as the interfering substance, effects such as follows can be expected:
1) elongated retention time in blood through the suppressed degradation of the interfering substance due to its large molecular weight; and
2) enhanced affinity for target molecules due to dimerization of the interfering substance.

When the receptor is a heterologous protein to the host, In vivo stability of the receptor is expected to be enhanced by its chimerization with a host protein. This fusion substance can be introduced into the living body by expressing a chimeric gene encoding the fusion substance.

An interfering substance is preferably derived from an organism which is the subject of administration. Therefore, when the method of the present invention is applied to humans, a protein derived from human is preferably used. Using a protein derived from the same organism, a safer formulation that avoids induction of the attack by the immune mechanism can be obtained. Furthermore, such interfering substance would not be eliminated through the immune mechanism, and thus effective administration can be expected.

When an antibody or a fragment thereof is used as an interfering substance, its safety can be enhanced by substituting a part thereof with a structure of human immunoglobulin. Specifically, a part of immunoglobulins derived from non-human species can be humanized by techniques such as chimeric antibody production and complementarity determining region (CDR) grafting. A humanized protein obtained in this manner is preferable as the interfering substance when the present invention is applied to humans.

In accordance with the observed overexpression of the gene product, an interfering substance of the present invention is administered into a site where the gene product is present. For example, when overexpression of the gene product is observed in blood, the interfering substance is administered orally or parenterally so that it can be present in blood in an effective amount to regulate the activity of the initial gene product. The administration method is not particularly limited. Increased retention time in blood of the interfering substance of the present invention can be expected by administering it as a binding product with a polymerized compound. The polymerized compound may include hyaluronic acid, polyethylene glycol, albumin, and such.

In the present invention, gene therapy can be used to administer an interfering substance consisting of a protein. Specifically, DNA encoding the interfering substance can be integrated into a known vector for administration into living body. A protein, the interfering substance, derived from the same specie as that to which the gene is administered express the interfering substance in the living body is safe, because it does not stimulate the immune mechanism. Furthermore, an accurate and continuous effect of the interfering substance can be obtained due to its sustained production in the living body without being attacked by the immune mechanism'. Moreover, a safe preparation for gene therapy can be produced inexpensively with smaller production facilities than industrially producing and purifying proteins.

For *in vivo* coexistence of an interfering substance by administration and expression of a DNA encoding it, the interfering substance is preferably expressed so that it is provided to a site where a protein that should be regulated by theinterfering substance is present. For example, when regulating the activity of a protein in blood, the interfering substance can be expressed as a secretory protein. Herein, the interfering substance expressed as a secretory protein is referred to as "soluble protein".

When a membrane protein, such as a receptor, is used as an interfering substance, its ligand-binding region can be expressed as a secretory protein. More specifically, when an EPO receptor is used for EPO, DNA encoding an amino acid sequence comprising the N-terminal amino acid residues at position 1 to 222 but not the transmembrane region of the EPO receptor can be used. A secretion signal is further added to the N-terminus of a protein consisting of such amino acid sequence to express it as a secretory protein. For example, secretion signal and ligand-binding region of the human EPO receptor is included within position 1 to 246 of the precursor protein encoded by cDNA of the human EPO receptor. The secretion signal added to the N-terminus can be an amino acid sequence derived from either the EPO receptor or other species that can function *in vivo.* Herein, the EPO receptor expressed as a secretory protein as described above is referred to as "soluble EPO receptor".

A DNA encoding an interfering substance can be introduced into a living body by direct administration of a virus vector or DNA. Many virus vectors, such as those using retrovirus, lentivirus, adenovirus, and adeno-associated virus (AAV) have been developed. "The direct administration of DNA" is a method wherein a viral structure-free expression vector for eucaryotic cells is administered into a living body *in vivo, in situ,* or ex *vivo.* Many methods have been performed for artificially introducing foreign genes into a living body. The present invention can be applied to .expression products of any gene artificially introduced by known gene introduction methods.

Specifically, vectors shown below may be used for the purpose of introducing a gene into a living body. Any of these vectors can be used for the introduction of DNA encoding an interfering substance of the present invention.
Plasmid expression vectors: pVR, pCMV, and pCAGGS
Duplication defective adenoviruses: AdEF1 and AdMLP
Retrovirus vector: LrEPSN
Adeno associated virus vectors: rAAV-ET and AdCMV

Among these various vectors, virus vectors such as retrovirus, adenovirus, and adeno-associated virus may cause a phenomenon wherein other cells are infected with the introduced vector. This phenomenon, called the "bystander effect", results in sustained expression of an introduced gene and thus is effective for obtaining strong expression. However, the introduction of an interfering substance in the present invention is intended to regulate the activity of an expression product of a gene introduced for therapy. Therefore, sustained expression over the expected level may act competitively with the therapeutic effect.

However, by adopting an inducible promoter for regulating the expression of an interfering substance, a virus vector can be utilized in the present invention. Specifically, the expression of the interfering substance can be regulated by administering a specific drug utilizing a promoter whose expression is induced by the drug. The use of such a virus vector, even if continuous infection of the vector occurs, can prevent unnecessarily high suppression of the activity of a gene expression product to be regulated by the interfering substance.

Thus, gene therapy using an expression vector, consisting exclusively of minimum elements including an expression regulatory region and a gene to be introduced, is a preferable method for introducing DNA encoding an interfering substance of the present invention.

A general expression vector is composed of expression regulatory regions, such as a replication origin, promoter, enhancer, and polyadenylating sequence; a foreign gene to be expressed; and so on. A method using such expression vectors in place of a virus vector is called the "naked DNA method". Herein, a "naked DNA" is defined as an expression vector consisting exclusively of DNA.

Therefore, a naked DNA is free of biological structures, such as proteins and sugars , that are observed in virus vectors. According to the naked DNA method, a gene is transiently expressed, no spontaneous intercellular infection of a vector (bystander effect) occurs, and the method implies no risk that the vector may be integrated into the host chromosome; which makes the method preferable for the present invention. Furthermore, the naked DNA is advantageous in that a larger amount of the vector can be prepared at lower costs as compared with viral vectors . Any of these methods for introducing a gene can be used as the method for introducing DNA encoding an interfering substance of the present invention.

To utilize the naked DNA method for expressing an interfering substance of the present invention, for example, an expression vector as follows is constructed. Specifically, the expression vector is composed of a DNA encoding the interfering substance, a promoter region, a 3'-region defining a transcription termination signal, a polyadenylating region, and so on. The promoter region should function in the cell or living body targeted to express the DNA encoding the interfering substance.

The promoter region may be derived from, for example, other organisms or other genes so long as it allows expression of the DNA encoding an interfering substance. For example, promoters derived from other eucaryotic genes or viral genes may be used. So long as the promoter can express the DNA encoding the interfering substance, the promoter does not have to be specific . When an inducible promoter is used, the expression thereof can be regulated by the induction condition.

Alternatively, when a promoter having tissue specificity is used, an interfering substance can be expressed specifically in a certain organ. Accordingly, such promoter enables application of the method for expressing the interfering substance in a tissue which is actually damaged by the overexpression of a gene expression product. For example, when the oxygen supply in a tissue is saturated due to overexpression of EPO, an artificial feedback loop can be constructedby integrating a promoter region responding to high oxygen concentration upstream of the introduced gene encoding the interfering substance. Specifically, promoters of following genes can be used.

Non-specific promoters:
Chicken β-actin, smooth muscle α-actin, thymidine kinase, metallothionein, interferon, and immunoglobulin

Inducible promoters:
steroid hormone receptors

Virus-derived promoters:
SV40, hepatitis B virus, and adenovirus major late gene

The promoter may be modified by introduction of a nucleotide sequence constituting another expression regulatory region. For example, the gene transcription efficiency of the chicken β-actin promoter is known to be improved by modifying a part thereof with rabbit (β-globulin gene-derived splicing acceptor (Japanese Patent No. 2824434 (1998)).

The expression vector may contain a terminator and an enhancer, in addition to a promoter. These expression regulatory regions can be derived not only from the gene to be introduced, but also from other genes or other species, as is the case with the promoter.

A naked DNA capable of expressing DNA encoding an interfering substance can be introduced into a living body by known methods. For example, *ex vivo* introduction of a DNA is performed by transplanting skin cells or blood cells introduced with an expression vector into a living body. When skin cells are transplanted, the expression level of the interfering substance can be easily regulated by removing the skin cells. The introduction of the expression vector into cells can be conducted by the electroporation method or such. The introduction of the naked DNA into the living body by electroporation is carried out, for example, in the following manner. Specifically, after intramuscular injection of the DNA into the limb, electrodes are set so that the injected site is sandwiched between them, and voltage is applied by pulsing according to the electroporation method. To introduce a naked DNA into an organ transplant, the DNA can be efficiently introduced with less damage to tissues by the electroporation method wherein the organ injected with the DNA is soaked in a solution, such as physiological saline, and the whole solution is stimulated with pulses without contacting the organ directly with electrodes. Alternatively, a DNA encoding the interfering substance can be expressed in organs, such as liver and kidney, by intravenous administration according, to the hydrogel method. The gene introduction by intravenous administration using TransIT In Vivo " Gene Delivery System (Mirus Corporation) or such can also be used.

Moreover, for introducing a DNA encoding an interfering substance, the hemagglutinating virus of Japan (HVJ)-liposome method having both characteristics of easily-handled liposome method and highly-efficient virus vector method may be used. According to this method a DNA can be efficiently introduced and expressed in even nondividing cells, and the DNA is expressed transiently without being introduced into the host genome. Accordingly, it can be said that, similar to the naked DNA, this method is a preferable expression system for the DNA encoding the interfering substance of the present invention.

An introduced DNA as described above encoding an interfering substance is expressed in introduced cells to produce the interfering substance *in vivo*. The produced interfering substance suppresses the activity of an expression product of an artificially introduced gene. The expression of the interfering substance is reduced and disappears in accordance with the life span of the transformed cells, thus does not unnecessarily reduce the activity of the expression product of the artificially introduced gene.

### Brief Description of the Drawings

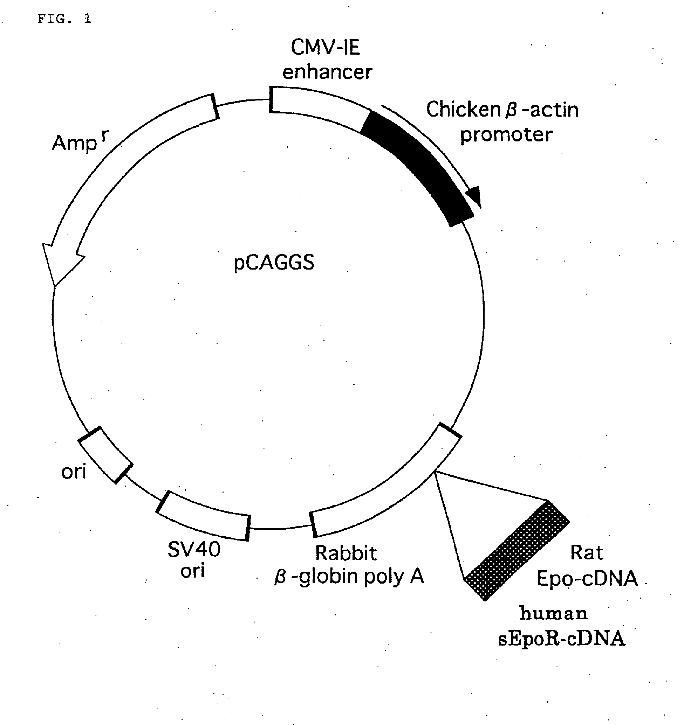

Fig. 1 is a schematic illustration showing the structure of expression vectors of the present invention.

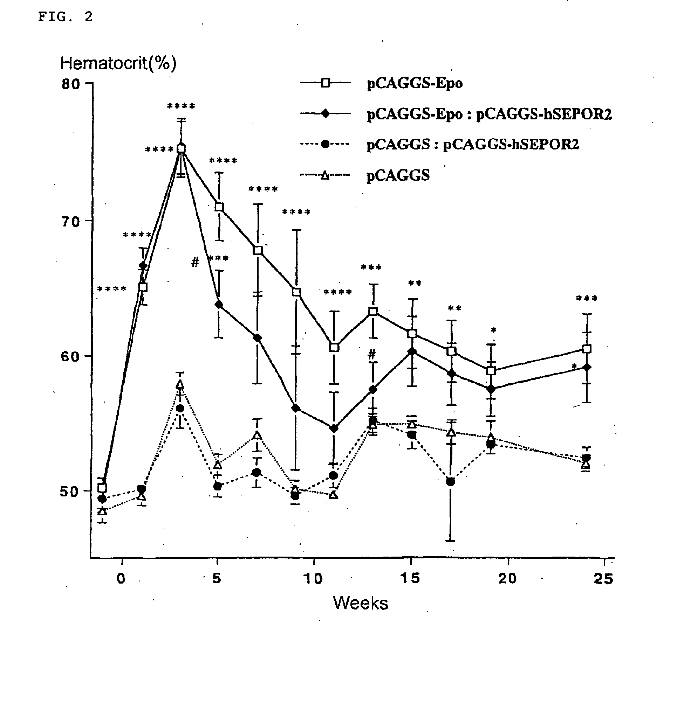

Fig. 2 is a graph showing the changes in the hematocrit value in blood of rats introduced with pCAGGS-EPO and pCAGGS. The hematocrit value (%) is shown on the vertical axis, and the time course (weeks) after the introduction of pCAGGS-EPO is shown on the horizontal axis. In the graph, * : p<0.05, **: p<0.01, ***: p<0.001, and *^{***}: p<0.0001.

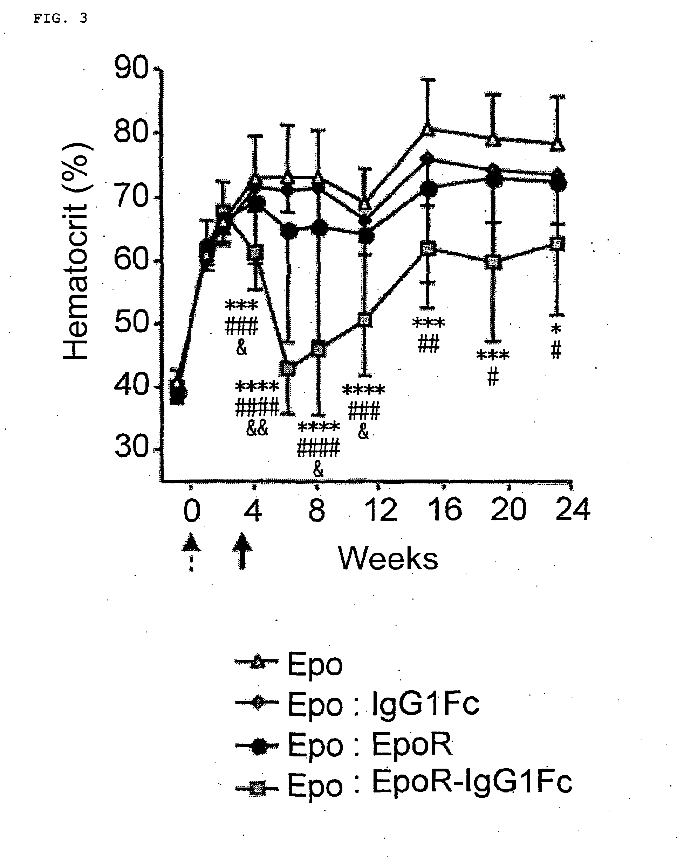

Fig. 3 is a graph showing the changes in the hematocrit level in blood. The hematocrit value (%) is shown on the vertical axis, and the time course (weeks) on the horizontal axis. In each group, n= 9. In the graph, *: p<0.05, ***: p<0.001, ****: p<0.0001, #: p<0.05, ##: p<0.01, ###: p<0.0001, ####: p<0.0001, &: p<0.05, &&: p<0.001.

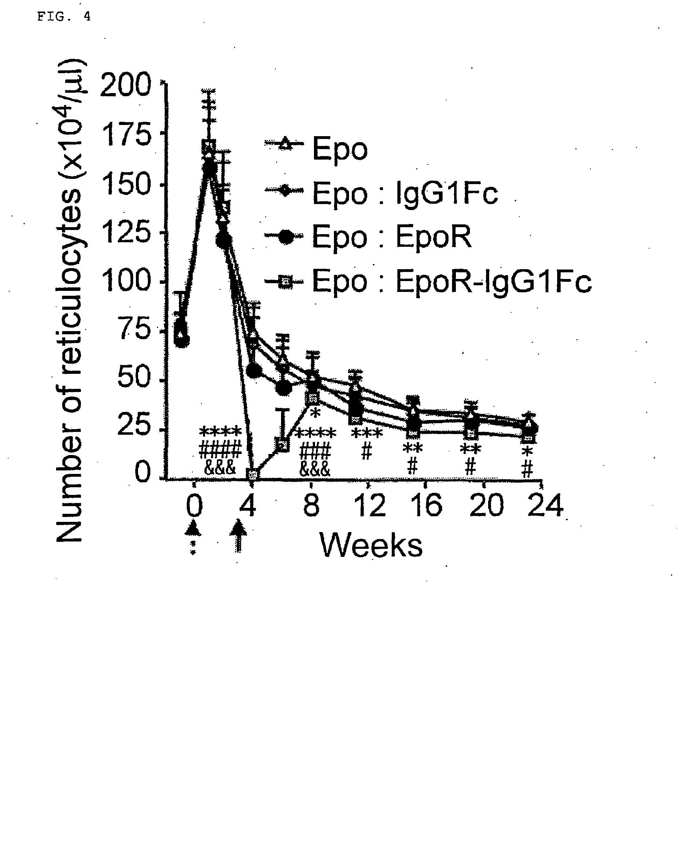

Fig. 4 is a graph showing the changes in the number of reticulocytes in blood. The number of reticulocytes (x10⁴/µl) is shown on the vertical axis, and the time course (weeks) on the horizontal axis. In each group, n = 9. In the graph, *: p<0.05, **: p<0.01, ***: p<0.001, ****: p<0.0001, #: p<0.05, ###: p<0.0001, ####: p<0.0001, &&&: p<0.001.

### Best Mode for Carrying out the Invention

This invention is specifically illustrated below with reference to Examples, but it is not to be construed as being limited thereto.

The animals used in the Examples were 8-weeks-old male rats (SD, Charles River Inc. , Tokyo) kept for one week or more in a light-dark (each 12 hours) cycle under pathogen-free conditions before starting the experiment. All the animals were supplied with water and standard food (MF containing 23.8% protein; 0.24% sodium; 0.0154% iron, Oriental Yeast Co., Ltd., Tokyo) ad libitum.

### [Example 1] Preparation of plasmid vectors:

Plasmid pCAGGS-Epo was constructed by inserting rat EPO cDNA into the XhoI site of expression vector pCAGGS (Niwa H et al., Gene, 1991, 108, 193-199). Plasmid pCAGGS-hSEPOR2 was constructed by inserting a cDNA of human soluble EPO receptor (hSEPOR2 , WO 99/53313) into the XhoI site of the expression vector pCAGGS in the same manner as described above. Each plasmid was purified by Qiagen EndoFree plasmid Giga kit (Qiagen GmbH, Hilden, Germany) (Maruyama H et al., Human Gene Therapy, 2000, 11, 429-437). The structure of the expression vectors constructed in this Example is shown in Fig. 1. The nucleotide sequences encoding the human soluble EPO receptor and the rat EPO are described in SEQ ID NOs: 1 and 2, respectively.

As the control, plasmid pCAGGS was used. The resulting DNA was dissolved in sterilized phosphate-buffered saline (PBS) and prepared to a concentration of 2 µg/µl for the injection.

### [Example 2] Introduction of pCAGGS-Epo and pCAGGS:

Intramuscular injection of the plasmid DNA by the electroporation method was conducted according to previously-reported method (Maruyama H et al., Human Gene Therapy, 2000, 11, 429-437). Specifically, fifty µg of plasmid DNAs, pCAGGS-Epo and pCAGGS, were inj ected into both the medial and lateral sites of the right and left lower limbs (200 µg in total) , respectively. Then, a pair of stainless steel electrodes of 10 mm width and 5 mm length were set so that the DNA-injected site is located in the center of the two electrodes, and electric pulse generator (Electro Sequre Porator T820; BTX) was used to give electricpulses (8 pulses, duration 50 msec, 100 V, 1 pulse/s).

After the introduction of the gene, the animals were lightly anesthetized with diethyl ether, 2 ml blood was collected from the heart according to the method of Ohwada et al. (Ohwada K. , Exp. Anim. , 1986, 35, 353-355), and the EPO level in serum was measured using Recombigen EPO kit (Nippon DPC, Chiba) . The erythrocyte, hematocrit, hemoglobin, leukocyte, and platelet were measured with Sysmex SE-900 (Sysmex, Hyogo) and the reticulocyte with Sysmex R-3500.

The results are shown in Fig. 2. The experimental results are shown by mean ± standard error, and the statistically significant difference was evaluated by a t-test without correspondence, and p<0.05 was regarded as significant. Upon the introduction of the EPO gene, the hematocrit value increased and reached almost the peak after 3 weeks (Fig. 2). On the other hand, the group introduced with the control plasmid DNA showed an increase in the hematocrit value 4 weeks after the introduction, but the amount of increase was very slight as compared with that of the group introduced with the EPO gene.

### [Example 3] Introduction of pCAGGS-hSEPOR2:

The animals confirmed to have an increased hematocrit value in Example 2 were transformed with the human EPO soluble receptor expression vector (pCAGGS-hSEPOR2) on the fourth week. pCAGGS-hSEPOR2 was intramuscularly injected in the right and left tights in the same manner as in Example 2 at an amount of 800 µg in total. Electroporation and blood collection were also conducted in the same manner as in Example 2.

The hematocrit value of the group introduced with the human EPO soluble receptor expression vector tended to decrease compared with that of the group without introduction, and a significant reduction in the hematocrit value was confirmed on the fifth and thirteenth weeks. When the EPO soluble receptor was introduced to the control group after 4 weeks, the hematocrit value did not change thereafter to be further reduced, and caused no anemic conditions.

According to the results described above, excessive action of a gene introduced for therapeutic purpose can be reliably regulated by introducing a soluble receptor into the living body. Furthermore, at the time when the expression level of the introduced gene was reduced (on the 15 weeks or later in this experiment) or under normal conditions such as that shown in the control wherein the expression level of the foreign gene was zero, no strong side effects that may further lower the expression level was confirmed. Thus, the present method was considered to be a highly safe regulation method.

### [Example 4] Regulation of rat EPO levels by introducing human EPO receptor-IgG Fc chimeric gene:

In the above Examples, the action of rat EPO was suppressed by the human soluble EPO receptor. In this Example, changes in the action of rat EPO were detected using, in place of the human soluble receptor, a chimeric protein thereof with rat IgGFc.

IgG1Fc cDNA was cloned from commercially-available rat spleen library (Quick Clone, Clontech) by the PCR method. The IgGlFc cDNA was inserted into the expression vector pCAGGS in the same manner as in Example 1 to construct rat IgGFc expression plasmid pCAGGS-IgG1Fc In addition, a chimeric cDNA, IgGlFc cDNA ligated with hEPOR, was inserted into the XhoI site of the expression vector pCAGGS in the same manner as in Example 1 to construct chimeric protein expression plasmid pCAGGS-hSEPOR2-IgG1Fc. The nucleotide sequence of the insert in the chimeric protein expression plasmid pCAGGS-hSEPOR2-IgG1Fc is shown in SEQ ID NO: 3. Each plasmid was purified as in Example 1 and used in the following experiment.

100 µg pCAGGS-Epo was intramuscularly injected by the electroporation method in the medial and lateral sites of the right and left lower limbs of an animal (rat) (400 µg in total) . pCAGGS-Epo is a plasmid expressing rat Epo. One and two weeks after the gene introduction, increase in EPO levels in blood were confirmed, respectively, and 3 weeks later, 800 µg of each plasmid, pCAGGS-IgG1Fc, pCAGGS-hSEPOR2, and pCAGGS-hSEPOR2-IgG1Fc, were introduced via a tail vein into the rat using *Trans*IT® *In Vivo* Gene Delivery System (MIR5125, Mirus) (Liu F, Song YK, Liu D, Hydrodynamics-based transfection in animals by systemic administration of plasmid DNA, Gene Therapy, 1999, 6, 1258-1266).

Blood was collected in the course of time, and the EPO level, the hematocrit value, and the number of reticulocytes in blood were measured for the following 24 weeks. For statistically significant difference test, t-test without correspondence was carried out, and level of significance of 5% or less was regarded as significant.

Animals with increased EPO levels upon the introduction of the plasmid pCAGGS-hEPO were grouped such that each group had a similar degree of increase, and each of the soluble human EPO receptor was introduced via a tail vein. As a result, the serum EPO level reached the peak after 1 week due to the introduction of EPO, and then gradually decreased (data not shown). The hematocrit value increased immediately after the introduction, reached a peak after 4 weeks, and then remained almost constant. Thereafter, though not significant to the value of the initial peak, the level increased again after 16 weeks and this increased level was maintained even after 24 weeks (Fig. 3).

On the other hand, the group introduced with the hSEPOR2-IgG1Fc chimeric protein expression plasmid showed a significant decrease in the hematocrit value from the fourth week to the twenty-fourth week, as compared with that of the group introduced with EPO (Fig. 3) . The group into which IgGlFc or soluble human EPO receptor had been introduced showed a tendency to lower the hematocrit value as compared with the group introduced with the EPO alone, but their difference was not significant. The action of the hSEPOR2-IgG1Fc chimeric protein was also significant compared with the IgGlFc introduced group, and also showed a significantly stronger effect from the fourth week to the twelfth weeks in comparison with the soluble human EPO receptor introduced group (Fig. 3).

The number of reticulocytes, reached the peak one week after the introduction of EPO and rapidly decreased thereafter. No change was observed in the reticulocyte level by the introduction of IgG1Fc or EPO receptor as compared with the group introduced with the EPO gene alone. However, a significant decrease in the number of reticulocytes from the fourth week to the twenty-fourth week was observed by the introduction of the hSEPOR2-IgG1Fc chimeric protein. This action was significantly stronger than that in the groups introduced with IgF1Fc and EPO receptor, respectively (Fig. 4).

As a result, increased EPO level in blood is more strongly reduced by introducing the chimeric receptor with rat IgG1Fc (hSEPOR2-IgG1Fc than by expressing the EPO receptor alone.

The introduction of the EPO receptor in Example 2 (Fig. 2) significantly reduced the increased hematocrit value. However, in Example 4 (Fig. 3), despite the same tendency was observed the difference was not significant. This difference in the effect of the EPO receptor was ascribed to the difference in the introduced amount of rat Epo (pCAGGS-EPO). The introduced pCAGGS-EPO amount was 200 µg in Example 2 (Fig. 2), while it was 400 µg in Example 4 (Fig. 3) . That is, the same amount of EPO receptor as in Example 2 may have failed to sufficiently neutralize the activity of EPO in Example 4, due to the fact that the EPO is expressed at a larger amount in Example. 4 than in Example 2.

### Industrial Applicability

This invention realizes a method for regulating the activity of an expression product of an introduced gene *in vivo,* which was unconsidered in conventional gene therapy. Constant expression of a gene artificially introduced into a living body at a higher level than the physiological level can cause side effects *in vivo*. For example, when the introduced gene encodes EPO, it causes side effects such as plethora.

According to the present invention, side effects caused by overexpression can be reduced by administering an interfering substance. The Examples exemplify the administration of the interfering substance via the introduction of a gene encoding a soluble receptors Apart from receptors, similar effects can be achieved by introducing a neutralizing antibody or a gene encoding the antibody.

The method of administering a recombinant soluble receptor protein or an antibody protein, however, requires a large amount of the purified proteins and repetitive administration to achieve an inhibitory action, which increases cost and physical burden, such as regular treatment in the hospital, on patients. The method of introducing a gene demonstrated by the present inventors is excellent in terms of the durability of the effect and safety, since the protein is produced in cells of tissues of the patient himself and thus has less antigenicity with respect to sugar chain modification and such. Furthermore, when the gene isintroduced into the skin, the introduced gene can be easily switched on/off by transplanting the skin.

## Claims

1. A method for regulating the activity of an expression product of a gene introduced into a living body involving the coexistence of a protein that interferes with the activity of the expression product.

2. The method according to claim 1, wherein the coexistence of the protein that interferes with the activity of the expression product is achieved by expressing a gene encoding the protein in the same living body.

3. The method according to claim 2, wherein the gene encoding the protein that interferes with the activity of the expression product is introduced into the living body via an expression vector consisting of a naked DNA.

4. The method according to claim 3, wherein the expression vector is introduced into the living body by electroporation.

5. The method according to claim 1, wherein the protein that interferes with the activity of the expression product is a protein selected from the group consisting of: a receptor of the expression product, an activity-neutralizing antibody of the expression product, and a protein containing an active site of the receptor or the antibody.

6. The method according to claim 2, wherein the gene encoding the protein that interferes with the activity of the expression product is expressed under the control of an inducible promoter.

7. A method for regulating the activity of an expression product of a gene introduced in gene therapy by the method according to claim 1.

8. The method according to claim 7, wherein the introduced gene encodes a protein which belongs to a group selected from blood proteins, cytokines, and hormones.

9. The method according to claim 8, wherein the protein belonging to a group selected from blood proteins, cytokines, and 'hormones is selected from the group consisting of:
erythropoietin, granulocyte-colony stimulating factor, granulocyte-macrophage colony stimulating factor, stem cell factor, thrombopoietin, tumor necrosis factor-α, transforming growth factor-β, CD4, CD45, bone morphogenetic protein, interferon-α, interferon-γ, interleukin-1, interleukin-2, interleukin-6, interleukin-11, interleukin-12, epidermal growth factor, acidic fibroblast growth factor, basic fibroblast growth factor, fibroblast growth factor-I, platelet derived growth factor, vascular endothelial growth factor, nerve growth factor, brain derived neurotrophic factor, insulin-like growth factor-I, insulin-like growth factor-II, human growth hormone, parathyroid hormone, angiostatin, pigment epithelial cell derived factor, and hepatocyte growth factor.

10. The method according to claim 9, wherein the protein is erythropoietin.

11. The method according to claim 10, wherein the protein that interferes with the activity of the expression product is a solubilized erythropoietin receptor.

12. The method according to claim 1, wherein the protein that interferes with the activity of the expression product is a fusion protein with another protein.

13. An expression vector for regulating the activity of an expression product of a gene introduced into a living body, which can express a protein that interferes with the activity of the expression product in the same living body.

14. The expression vector according to claim 13, wherein the protein that interferes with the activity of the expression product is a fusion protein with another protein.

15. A composition for regulating the activity of an expression product of a gene introduced into a living body, which comprises the expression vector of claim 13 as an active ingredient.

16. Use of an expression vector that can express a protein which interferes with the activity of an expression product of a gene introduced into a living body in the same living body, in the production of a composition for regulating the activity of the expression product of a gene introduced into a living body..

17. The use of the expression vector according to claim 16, wherein the protein that interferes with the activity of the expression product is a fusion protein with another protein.
